# EUROPEAN PATENT SPECIFICATION

(11) **EP 0 909 752 B1**
(45) Date of publication and mention of the grant of the patent: **05.09.2001**
(21) Application number: 98905733.6
(22) Date of filing: 03.03.1998
(51) Int. Cl.: C07C 69/54

(54) **DI(METH)ACRYLATES**
DI(METH)ACRYLATE
DI(METH)ACRYLATES

(30) Priority: 04.03.1997 JP 4863397
(43) Date of publication of application: 21.04.1999
(73) Proprietor: Kyowa Yuka Co., Ltd., Tokyo 100-0004 (JP)
(72) Inventor: MUTO, Kenji, Yokkaichi-shi, Mie 512-0911 (JP); MURAYAMA, Toshikazu, Yokkaichi-shi, Mie 512-0911 (JP); TSUZAKI, Nobuko, Yokkaichi-shi, Mie 512-0911 (JP)
(74) Representative: Le Guen, Gérard
(86) International application number: JP9800862
(87) International publication number: WO9839285

(56) References cited:
- JP-A- 9 251 157
- JP-A- 63 250 346
- US-A- 3 544 536

## Description

The present invention relates to a di(meth)acrylate which is useful for UV light or electron ray curing coating agents, coating materials, printing ink, adhesive agents, or as diluents therefor.

### Background Art

There have been remarkable advance in the electronic materials field in recent years, giving rise to a substantial market for optical disks, optical fiber, optical magnetic disks and the like. Study has been directed to UV light or electron ray curing materials for the binders, adhesive agents or coating agents that are employed in the electronic materials field. However, conventionally known acrylic esters, such as 1,6-hexanediol diacrylate, neopentylglycol diacrylate, and trimethylolpropane triacrylate, are problematic with respect to their water resistant properties. Thus, when considering practical applicability, the long term stability of these products has not been considered satisfied.

In addition, 1,6-hexanediol diacrylate, neopentylglycol diacrylate, and trimethylolpropane triacrylate are also problematic due to their strong stimulatory effect on the skin.

Japanese Published Unexamined Patent Application No. 25 0346/88 discloses 3-methyl-1,5-pentanediol dimethacrylate and the like, and Japanese Published Unexamined Patent Application No. 66643/91 discloses 2-butyl-2-ethyl-1,3-propanediol dimethacrylate and the like. However, neither of these dimethacrylate products demonstrates satisfactory toughness and flexibility for practical applications.

### Disclosure of the Invention

The present invention provides a di(meth)acrylate, i.e., a diacrylate or a dimethacrylate, represented by the following general formula (I): 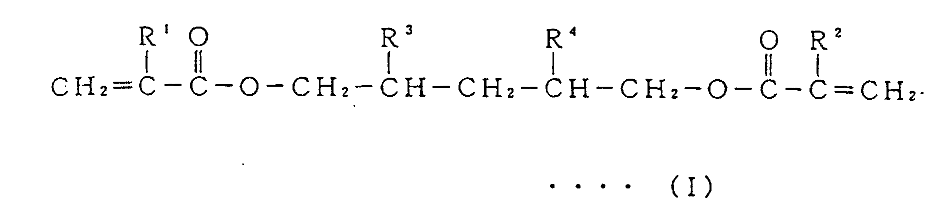 (wherein R¹ and R² are the same or different and represent a hydrogen atom or CH₃; and R³ and R⁴ are the same or different and represent lower alkyl of 1-6 carbon atoms).

In the definitions of the group in general formula (I), lower alkyl of 1-6 carbon atoms R³ and R⁴ means straight-chain or branched alkyl group, examples of which include methyl, ethyl, propyl, isopropyl, butyl, isobutyl, tert-butyl, pentyl, isopentyl, neopentyl, and hexyl. The di(meth)acrylate of the present invention does not have a stimulatory effect on the skin. In addition, by employing di(meth)acrylate of the present invention, it is possible to obtain a curing composition which is readily cured using UV, electron rays, heat or the like. The cross-linked cured product which is obtained from the curing composition gives an excellent toughness owing to good resistance to hydrolysis and is excellent in terms of flexibility.

### Modes for Carrying out the Invention

The di(meth)acrylate of the present invention can be prepared by (1) an esterification reaction between 2,4-dialkyl-1,5-pentanediol and (meth)acrylic acid, (2) an ester exchange reaction between 2,4-dialkyl-1,5-pentanediol and a (meth)acrylic acid ester such as methyl (meth)acrylate or ethyl (meth)acrylate, or (3) a reaction between 2,4-dialkyl-1,5-pentanediol and (meth)acryloyl halide like (meth)acryloyl chloride.

Specific examples of the starting material, 2,4-dialkyl-1,5-pentanediol include 2,4-dimethyl-1,5-pentanediol, 2-ethyl-4-methyl-1,5-pentanediol, 2-methyl-4-propyl-1,5-pentanediol, 2-isopropyl-4-methyl-1,5-pentanediol, 2,4-diethyl-1,5-pentanediol, 2-ethyl-4-propyl-1,5-pentanediol, 2-ethyl-4-isopropyl-1,5-pentanediol, 2,4-dipropyl-1,5-pentanediol, 2-isopropyl-4-propyl-1,5-pentanediol, 2,4-diisopropyl-1,5-pentane diol, 2,4-dibutyl-1,5-pentanediol, 2,4-dipentyl-1,5-pentanediol and 2,4-dihexyl-1,5-pentanediol.

2,4-Dialkyl-1,5-pentane diol can be produced using a known method. A preferred approach as disclosed in Japanese Published Unexamined Patent Application No. 48642/96 or in EP807617A is to react formaldehyde with a 2-butenal derivative, and then hydrogenate the obtained reaction product.

The esterification reaction between 2,4- dialkyl-1,5-pentane diol and (meth)acrylic acid described in (1) above will now be explained.

The amount of (meth)acrylic acid employed is about 2-10 moles per 1 mole of 2,4-dialkyl-1,5-pentanediol. The esterification reaction between 2,4-dialkyl-1,5-pentanediol and (meth)acrylic acid is generally carried out at about 60∼140°C, and preferably at 75∼100°C.

A catalyst may be employed in the esterification reaction between 2,4-dialkyl-1,5-pentanediol and (meth)acrylic acid, suitable examples thereof including acid catalysts such as sulfuric acid, p-toluenesulfonic acid, and phosphoric acid; metal oxides such as zinc oxide and germanium oxide; and esterified catalysts such as tin octylate and tetrabutyl titanate. Generally, the concentration of the catalyst is in the range of 0.001-5 wt% of the reaction mixture.

In order to efficiently remove the water generated in the esterification reaction, an azeotropic solvent may be employed. Examples include hexane, cyclohexane, benzene, toluene, xylene, and mixtures thereof.

After the completion of the reaction, the target product may be purified using methods conventionally employed in synthetic organic synthesis chemistry, such as filtration, extraction, washing, drying, concentrating or distillation. For example, the product may be purified by separating the organic layer using filtering, neutralizing the unreacted (meth)acrylic acid, and acid catalyst using an alkaline aqueous solution such as sodium hydroxide, washing with water , and then distilling off the solvent under reduced pressure.

The ester exchange reaction between 2,4-dialkyl-1,5-pentane diol and a (meth)acrylic acid ester like methyl (meth)acrylate or ethyl (meth)acrylate described in (2) above will now be explained.

The reaction and purification conditions for the ester exchange reaction are the same as those described in (1) above. In addition, the reaction may also be carried out under reduced pressure.

The reaction between 2,4-dialkyl-1,5-pentanediol and a (meth)acryloyl halide described in (3) above will now be explained.

The amount of (meth)acryloyl halide employed is about 2-4 moles per 1 mole of 2,4 dialkyl-1,5-pentanediol. The reaction between the (meth)acryloyl halide and 2,4-dialkyl-1,5-pentanediol is generally carried out at about 150°C or less, and preferably at 100°C or less. A tertiary amine, such as triethylamine, pyridine or the like, may be employed as the catalyst. By employing such catalysts, the reaction proceeds efficiently in a short period of time. Examples of the reaction solvent include hexane, cyclohexane, benzene, toluene, xylene and the like, as well as mixtures thereof.

After the completion of the reaction, the desired product may be obtained by washing with an alkaline aqueous solution followed by washing with water, and then purifying by removing the solvent or distilling.

In order to control double bond polymerization derived the (meth)acrylic acid during the reaction, the above-described reactions (1)∼(3) are preferably carried out in the presence of a polymerization inhibitor.

The polymerization inhibitor is used preferably at a concentration of 0.1-5 wt% based on the reaction mixture. As the polymerization inhibitor, hydroquinone, hydroquinone monomethyl ether, 2,4-dimethyl-6-t-butylphenol, or phenothiazine, may be employed.

A preferable example of the di(meth)acrylate of the present invention is a di(meth)acrylate wherein R¹ and R² in general formula (I) are the same, suitable examples of which include 2,4-dimethyl-1,5-pentanediol di(meth)acrylate, 2-ethyl-4-methyl-1,5-pentanediol di(meth)acrylate, 2,4-diethyl-1,5-pentanediol di(meth)acrylate, 2-ethyl-4-propyl-1,5-pentanediol di(meth)acrylate, 2,4-dipropyl-1,5-pentanediol di(meth)acrylate, 2-isopropyl-4-methyl-1,5-pentanediol di(meth)acrylate, 2-ethyl-4-isopropyl-1,5-pentanediol di(meth)acrylate, 2,4-diisopropyl-1,5-pentanediol di(meth)acrylate, and 2-isopropyl-4-propyl-1,5-pentanediol di(meth)acrylate. Of these, 2,4-diethyl-1,5-pentanediol di(meth)acrylate is preferred.

By employing the di(meth)acrylate of the present invention, it is possible to obtain a UV, electron ray or heat curing composition. The compositions provide cross-linked cured products which have good resistant to hydrolysis and demonstrates excellent flexibility.

Thus, the di(meth)acrylate of the present invention can be employed as a UV light or electron ray curing coating agent, coating material, printing ink, adhesive agent, or a diluent therefor. When employed in these applications, the di(meth)acrylate of the present invention may be used as a composition with photo initiators , radical initiators, other acrylates, polymerization inhibitors, fillers, coloring agents, or wax according the object of the use. These compositions can readily be cured using electron rays, UV light or other irradiating rays, or by a heating means. For example, the composition may be coated on an iron, glass or wood substrate, irradiated with electron rays or UV light or other irradiating rays, or heated, to obtain a cured film. When curing with UV light, a photo initiator, such as a benzoin ether derivative such as benzoin isobutyl ether or benzoin isopropyl ether, or an acetophenone derivative such as α-hydroxyisobutylphenone, 2,2-dimethoxy-2-phenyl acetophenone, 1-hydroxycyclohexyl phenyl ketone, or phenothiazine, may be used singly or in combination. The amount of the photo initiator used is preferably in the range of 0.1∼10 wt% based on the composition. In heat curing, a radical initiator such as an organic peroxide or an azobis-derived compound may be used.

The di(meth)acrylate of the present invention may be employed in combination with other polyfunctional (meth)acrylates or copolymerizable oligomers and polymers.

Examples of other polyfunctional (meth)acrylates which may be employed together with the di(meth)acrylate of the present invention include trimethylolpropane triacrylate, dipentaerythritol hexacrylate or 1,6-hexanediol diacrylate.

Examples of the copolymerizable oligomer or polymer include diacrylates of poly(ethylene glycol) or poly(propylene glycol), diacrylates of ethylene oxide additives of bisphenol A, polyurethane acrylates or polyester acrylates, epoxy resin acrylates, polybutadiene acrylates, and resins containing unsaturated bonds such as unsaturated polyesters.

The amount of the di(meth)acrylate of the present invention contained in the composition may be determined according to the use. Preferably, the amount employed is about 3 parts by weight or more based on a total 100 parts by weight of the aforementioned polyfunctional (meth)acrylate and the di(meth)acrylate of the present invention.

### Examples

The present invention will now be explained by referring to Example.

### Example 1

144g of 2,4-diethyl-1,5-pentanediol, 195g of acrylic acid, 1.8g of sulfuric acid, 0.9g of hydroquinone monomethyl ether, and 270g of cyclohexane were placed in a 1 L reaction vessel equipped with a stirrer, thermometer, temperature adjusting device, condensor, and water separator. The temperature was increased, and the reaction was carried out at 85-90°C. The water produced due to azeotropy and the solvent were separated. The progress of the reaction was confirmed by gas chromatography under the analysis conditions mentioned below, and the reaction was conducted until the starting materials and mono-substituted acrylate were almost entirely exhausted.

After the completion of the reaction, cyclohexane was added, and the mixture was neutralized by washing with alkaline aqueous solution. Next, the mixture was washed with water twice, and the solvent was removed from the organic layer under reduced pressure, to obtain the product (2,4-diethyl-1,5-pentanediol diacrylate).

The obtained product had a viscosity of 11 cps (25°C), bromine value of 117, and saponification value of 417. The results of ¹H-NMR, ¹³C-NMR, and IR measurements on the obtained product were as follows.
IR (NaCl; cm⁻¹): 810, 968, 986, 1057, 1192, 1273, 1296, 1408, 1464, 1620, 1635, 1726, 2877, 2935, 2964
¹H-NMR (CDCl₃: ppm): 0.92 (6H, t, J=7.4Hz), 1.25∼1.45 (6H,m), 1.69-1.79 (2H,m), 4.07∼4.11 (4H,m), 5.80-5.84 (2H,m), 6.08-6.16 (2H,m), 6.37-6.42 (2H,m)
¹³C-NMR (CDCl₃: ppm): 166.4, 130.5, 128.6, 66.9, 36.4, 32.7, 24.3, 10.9

### (Conditions for gas chromatography analysis)

column filling agent: SE-30 (product of GL-SCIENCE Inc.)
column size: length 1.6 m, inner diameter 3.2 mm
detector: FID
injection temperature: 290°C
detector temperature: 290°C
column temperature: rising from 100°C to 290°C (10°C/min)
air flow volume: 50kP
H₂ flow volume: 60kP
N₂ flow volume: 40ml/min

### Example 2

144g of 2,4-diethyl-1,5-pentanediol, 248g of methacrylic acid, 1.8g of sulfuric acid, 0.9g of hydroquinone, and 270g of cyclohexane were placed in a 1 L reaction vessel equipped with a stirrer, thermometer, temperature adjusting device, condensor, and water separator. The temperature was increased, and the reaction was carried out at 87∼92°C. The water produced due to azeotropy and the solvent were separated. The progress of the reaction was confirmed by gas chromatography analysis, and the reaction was conducted until the starting materials and mono-substituted methacrylate were almost entirely exhausted.
After the completion of the reaction, cyclohexane was added, and the mixture was neutralized by washing alkaline aqueous solution. Next, the mixture was washed with water twice, and the solvent was removed from the organic layer under reduced pressure, to obtain the product (2,4-diethyl-1,5-pentanediol dimethacrylate). The obtained product had a viscosity of 9 cps (25°C), bromine value of 106, and saponification value of 377. The results of ¹H-NMR, ¹³C-NMR, and IR measurements on the obtained product were as follows.
IR (NaCl; cm⁻¹): 814, 939, 1013, 1169, 1321, 1377, 1462, 1639, 1720, 2877, 2931, 2962
¹H-NMR (CDCl₃: ppm): 0.92 (6H, t, J=7.4Hz), 1.27-1.45 (6H,m), 1.71-1.81 (2H,m), 1.94 (6H,s), 4.06∼4.10 (4H,m), 5.53-5.56 (2H,m), 6.08∼6.10 (2H,m)
¹³C-NMR (CDCl₃: ppm): 167.5, 136.5, 125.3, 67.0, 36.4, 32.8, 24.4, 18.3, 10.9

### Comparative Example 1

1,5-Pentanediol dimethacrylate was obtained by conducting the reaction substantially in the same manner as Example 2 except that 94g of 1,5-pentanediol was employed instead of 2,4-diethyl-1,5-pentanediol.

### Comparative Example 2

3-Methyl-1,5-pentanediol dimethacrylate was obtained by conducting the reaction substantially in the same manner as Example 2 except that 106g of 3-methyl-1,5-pentanediol was employed instead of 2,4-diethyl-1,5-pentanediol.

### Comparative Example 3

2-Butyl-2-ethyl-1,3-propanediol dimethacrylate was obtained by conducting the reaction substantially in the same manner as Example 2 except that 144g of 2-butyl-2-ethyl-1,3-propanediol propanediol was employed instead of 2,4-diethyl-1,5-pentanediol.

Table 1 show the viscosity (25°C) of the di(meth)acrylate synthesized in Examples 1 and 2 and Comparative Examples 1-3, and the viscosity (25°C) of commercial (meth)acrylate.

**Table 1:**

| Physical characteristics (viscosity) of various (meth)acrylic acid esters | | |
|---|---|---|
| | monomer | viscosity (25°C) |
| Example 1 | 2,4-diethyl-1,5-pentanediol diacrylate | 11 |
| Example 2 | 2,4-diethyl-1,5-pentanediol dimethacrylate | 9 |
| Comp. Ex. 1 | 1,5-pentanediol dimethacrylate | 5 |
| Comp. Ex. 2 | 3-methyl-1,5-pentanediol dimethacrylate | 6 |
| Comp. Ex. 3 | 2-butyl-2-ethyl-1,3-propanediol dimethacrylate | 15 |
| Commercially available product | 1,6-hexanediol diacrylate (Note 1) | 6 |
| Commercially available product | 1,6-hexanediol dimethacrylate (Note 2) | 6 |
| Commercially available product | trimethylolpropane triacrylate (Note 3) | 70 |
| Commercially available product | trimethylolpropane trimethacrylate (Note 4) | 43 |
| units of viscosity: cps | | |

| | | |
|---|---|---|
| Note 1: Light acrylate 1.6 HX-A, product of Kyoeisha Chemical Co., LTD. | | |
| Note 2: Light ester 1.6 HX, product of Kyoeisha Chemical Co., LTD. | | |
| Note 3: Biscoat #295, product of Osaka Organic Chemical Industries Co., LTD. | | |
| Note 4: Light ester TMP, product of Kyoeisha Chemical Co., LTD. | | |

### Test Examples 1∼6

5g of 2,2-dimethoxy-2-phenylacetophenone (product of Ciba-Geigy Japan Limited, IRUGACURE-651 was added as a photo initiator to 95g of each of the di(meth)acrylate synthesized in Examples 1-2 or Comparative Examples 1-3, and commercially available (meth)acrylate. After coating to a glass plate so as to form a film having a thickness of about 300-400 µm, the sample was irradiated with UV from a UV irradiation device (4 kw, 2 irradiation sources, irradiation distance of 20cm, on a conveyor at a speed of 2.5 m/min). The cured film was peeled from the glass plate to obtain a cured film having a non-sticky surface. Table 2 shows the appearance of the obtained cured film, results of bending tests, and the test results of the film's resistance to hydrolysis. The tests were carried out using the following methods.
Appearance of cured film: The inspection of cracking was confirmed with naked eyes.
Bending test: As a method for evaluating flexibility, the obtained film was bent and the film checked for the bending angle at which breakage occurred. A smaller bending angle indicates a greater degree of bending until the film breakage.
Resistance to hydrolysis (evaluation method): A 5% aqueous hydrochloric acid solution was placed in a glass vessel, and the cured film was immersed therein. After maintaining at 90°C for 10 days, the cured film was removed and the change (%) in weight from before to after the evaluation was checked.

**Table 2:**

| Test results on characteristics of cured film | | | | |
|---|---|---|---|---|
| | monomer | appearance of film | bending test | resistance to hydrolysis |
| Test Ex. 1 | 2,4-diethyl-1,5-pentanediol diacrylate (product synthesized in Ex. 1) | ○ | 10° | 3% |
| Test Ex. 2 | 2,4-diethyl-1,5-pentanediol dimethacrylate (product synthesized in Ex. 2) | ○ | 20° | 1% |
| Test Ex. 3 | 1,5-pentanediol dimethacrylate (product synthesized in Comp. Ex. 1) | X | 60° | 5% |
| Test Ex. 4 | 3-methyl-1,5-pentanediol dimethacrylate (product synthesized in Comp. Ex. 2) | X | 40° | 3% |
| Test Ex. 5 | 2-butyl-2-ethyl-1,3-propanediol dimethacrylate (product synthesized in Comp. Ex. 3) | △ | 100° | 1% |
| Test Ex. 6 | 1,6-hexanediol dimethacrylate (manufactured by Kyoeisha Chemical Co. LTD.) | X | 100° | 4% |
| Note - Appearance of film: The appearance of the cured film was evaluated as follows. no cracks ○ some cracks Δ numerous cracks X | | | | |

The cured film using the di(meth)acrylate of the present invention demonstrated excellent resistance to hydrolysis and had superior flexibility based on bending tests.

### Test Examples 7,8

The stimulatory effect on the skin of the di(meth)acrylate obtained in Examples 1 and 2 was checked using the following method. The results of these skin stimulation tests are shown in Table 3.

Skin stimulation tests (evaluation method): 1 ml of an aqueous solution formulated by diluting one part by weight of each of di(meth)acrylates with 100 parts by weight of ethanol was dripped onto gauze. The gauze was taped to the inside of the upper arm of a person. After one day, the gauze was removed, and the condition of the skin under the gauze was observed.

**Table 3:**

| Results of skin stimulation tests | | |
|---|---|---|
| | monomer | skin stimulation |
| Test Ex. 7 | 2,4-diethyl-1,5-pentanediol diacrylate (Note 1) | ○ |
| Test Ex. 8 | 2,4-diethyl-1,5-pentanediol dimethacrylate (Note 2) | ○ |
| (Note 1) product synthesized in Example 1 | | |
| (Note 2) product synthesized in Example 2 | | |
| (Note 3) Evaluation method: evaluation was as follows. ○ no change Δ red splotching present X redness, pain present | | |

The di(meth)acrylate of the present invention demonstrates only a low stimulatory effect on the skin.

### Industrial Applicability

Handling of the di(meth)acrylate of the present invention is facilitated due to its low stimulatory effect on the skin. A composition containing the aforementioned di(meth)acrylate is easily cured using UV light, electron rays, heat or the like, and provides a cured product with superior toughness and flexibility.

Accordingly, the di(meth)acrylate of the present invention is effectively employed as a UV light or electron ray curable coating agent, coating material, printing ink, adhesive agent, or as a diluent therefor. In particular, a composition containing the di(meth)acrylate of the present invention is optimally employed as a binder, adhesive agent or coating agent for optical disks, optical fiber, or optical magnetic disks.

## Claims

1. A di(meth)acrylate represented by the following general formula (I): 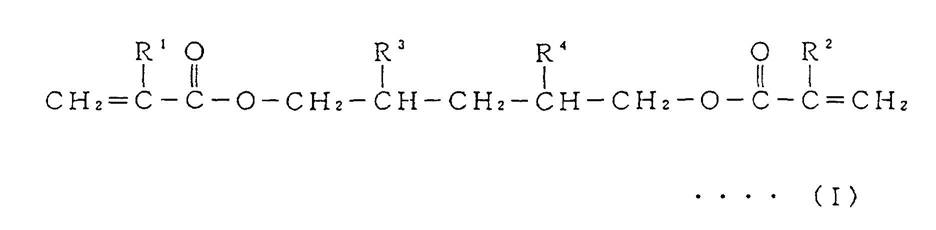 (wherein, R¹ and R² are the same or different and represent a hydrogen atom or CH₃, and R³ and R⁴ are the same or different and represent lower alkyl of 1-6 carbon atoms).

2. The di(meth)acrylate according to claim 1 in which R¹ and R² in general formula (I) are the same.

3. The di(meth)acrylate according to claim 1 or claim 2 in which R³ and R⁴ in general formula (I) are ethyl.

## Patentansprüche

1. Ein Di(Meth)Acrylat dargestellt durch die nachfolgende allgemeine Formel (I): 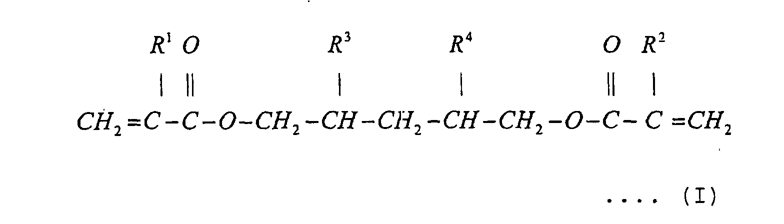 (in der R¹ und R² gleich oder verschieden sind und jeweils ein Wasserstoffatom oder CH₃ darstellen, und in der R³ und R⁴ gleich oder verschieden sind und jeweils niederes Alkyl mit 1 bis 6 Kohlenstoffatomen darstellen.)

2. Das Di(Meth)Acrylat gemäß Anspruch 1, bei dem R¹ und R² in der allgemeinen Formel (I) gleich sind.

3. Das Di(Meth)Acrylat gemäß Anspruch 1 oder 2, bei dem R³ und R⁴ in der allgemeinen Formel (I) Ethyl ist.

## Revendications

1. Di(méth)acrylate représenté par la formule générale (I) suivante : 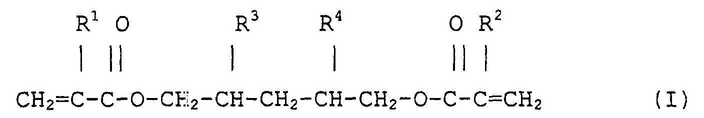 (dans laquelle R¹ et R² sont identiques ou différents et représentent un atome d'hydrogène ou un groupe CH₃, et R³ et R⁴ sont identiques ou différents et représentent un groupe alkyle inférieur comportant de 1 à 6 atomes de carbone).

2. Di(méth)acrylate selon la revendication 1, dans lequel R¹ et R² dans la formule générale (I) sont identiques.

3. Di(méth)acrylate selon la revendication 1 ou la revendication 2, dans lequel R³ et R⁴ dans la formule générale (I) représentent un groupe éthyle.
